# EUROPEAN PATENT APPLICATION

(11) **EP 4 437 887 A1**
(43) Date of publication of application: **02.10.2024**
(21) Application number: 22905973.8
(22) Date of filing: 15.09.2022
(51) Int. Cl.: A24F 40/485, A24F 40/10, A61M 15/06

(54) **ATOMIZER HAVING HOLLOWED-OUT ATOMIZATION COVER**

(30) Priority: 17.12.2021 CN 202111553335
(71) Applicant: Huizhou Happy Vaping Technology Limited, Huizhou, Guangdong 516000 (CN)
(72) Inventor: LIN, Guangrong, Shenzhen, Guangdong 518104 (CN); ZHENG, Xianbin, Shenzhen, Guangdong 518104 (CN)
(74) Representative: Hannke, Christian
(86) International application number: PCT/CN2022/119018
(87) International publication number: WO 2023/109220

(57) **Abstract**

A vaporizer with a hollow-out vaporizing cover part comprises a mouthpiece cap (1) and a vaporizer housing (2), the mouthpiece cap (1) is provided at an upper end with a mouthpiece hole (10), the vaporizer housing (2) has a mouthpiece end (21) and an opening end (22), and the mouthpiece cap (1) is sleeved on the mouthpiece end 21 of the vaporizer housing (2). The mouthpiece end (21) of the vaporizer housing (2) is closed and arranged at a center with a mouthpiece tube (210) extending downwards into the vaporizer housing (2). The vaporizer housing (2) is provided inside with a hollow-out vaporizing cover part (3), a tube-shaped vaporizing core (4), and a base (6). The hollow-out vaporizing cover part (3) is provided with a cavity (30), and a middle wall portion of the hollow-out vaporizing cover part (3) is hollowed out to define an opening (320). The cavity (30) is open to communicate with the liquid storage chamber (23) through the opening (320), the tube-shaped vaporizing core (4) is vertically arranged in the cavity (30) in such a manner that the tube-shaped vaporizing core (4) has an upper end connected with the hollow-out vaporizing cover part (3) and in communication with the mouthpiece tube (210), a lower end in communication with the first air inlet (60), and a middle outer wall of the tube-shaped vaporizing core (4) is entirely exposed to the cavity (30). It has advantages that, as the hollow-out vaporizing cover part (3) has the cavity (30) and the opening (320), the vaporizing liquid in the liquid storage chamber (23) can be quickly supplied to the surface of the tube-shaped vaporizing core (4), thereby avoiding dry-heating of the vaporizing core (4).

## Description

### TECHNICAL FIELD

The disclosure relates to the technical field of the vaporizer of electronic vaporizing device, and more particularly, the disclosure relates to a vaporizer with a hollow-out vaporizing cover part.

### BACKGROUND

Generally, an electronic vaporizing device includes a battery part and a vaporizer. A battery cell inside the battery part is to supply power to the vaporizer. The vaporizer includes a heating unit which can atomize the to-be-vaporized solution into vapor for users to vape when powered on. The electronic vaporizing device can be used as a vaporizing device for the electronic cigarette liquid, medicine liquid, herbal essence liquid, etc. Its basic task is to convert the solutions such as vaporizing liquid, medicine liquid, and the like stored in the electronic vaporizing device into vapor, aerosol, steam, etc.

Most vaporizers in the market are in the face of some technical difficulties. For example, the vaporizing liquid in the liquid storage chamber may not smoothly flow to the liquid absorbing surface of the vaporizing core due to the narrow passage between the liquid storage chamber and the vaporizing core. In the case that the vaporizing liquid cannot be timely fed to the vaporizing core, for example when the vaporizing liquid has a high viscosity, the heating element of the vaporizing core has a high power, or a large amount of vapor has been generated, dry-heating of the vaporizing core may occur. The material that is not vaporized in the vaporizing core may be charred at a high temperature, producing burning taste and bringing bad user experience. In addition, the heating element is prone to deformation and damage at a high temperature, thereby easily leading to malfunction.

### SUMMARY

### Technical problems

The disclosure aims to provide a vaporizer with a hollow-out vaporizing cover part, which has improved structure and thus allows quick feeding of the vaporizing liquid to the vaporizing core, thereby avoiding dry-heating of the vaporizing core.

### Solutions for the problems

### Technical solutions

A technical solution of the disclosure is as follows: A vaporizer with a hollow-out vaporizing cover part comprises a mouthpiece cap and a vaporizer housing, wherein the mouthpiece cap is provided at an upper end with a mouthpiece hole, the vaporizer housing has a mouthpiece end and an opening end, the mouthpiece cap is sleeved on the mouthpiece end of the vaporizer housing, and the mouthpiece end of the vaporizer housing is closed and is arranged at a center with a mouthpiece tube extending downwards into the vaporizer housing, and a hollow-out vaporizing cover part, a tube-shaped vaporizing core, and a base are arranged inside the vaporizer housing;
The base is sleeved on the opening end of the vaporizer housing, and the base is provided at a center with a first air inlet;
The hollow-out vaporizing cover part has an upper end connected with a lower end of the mouthpiece tube, a lower end of the hollow-out vaporizing cover part is open and is engaged to the base, and a liquid storage chamber for storing the vaporizing liquid is defined by a cavity formed between an inner wall of the vaporizer housing, an outer wall of the mouthpiece tube, and an upper surface of the hollow-out vaporizing cover part;
The hollow-out vaporizing cover part is provided inside with a cavity, a middle wall portion of the hollow-out vaporizing cover part is hollowed out to define an opening,

The cavity is open to communicate with the liquid storage chamber through the opening, the tube-shaped vaporizing core is vertically arranged in the cavity in such a manner that an upper end of the tube-shaped vaporizing core is connected with the hollow-out vaporizing cover part and in communication with the mouthpiece tube, a lower end thereof is in communication with the first air inlet, and the middle outer wall of the tube-shaped vaporizing core is entirely exposed to the cavity;
The first air inlet, the tube hole of the tube-shaped vaporizing core, the mouthpiece tube, and the mouthpiece hole are in communication in such order to define airflow paths which are sealed and separated from the liquid storage chamber.

Preferably, a seal seat may be further disposed between the tube-shaped vaporizing core and the base, the seal seat may seal the liquid storage chamber, the seal seat may be provided at a center with a second air inlet in communication with the first air inlet, and a lower end of the tube-shaped vaporizing core may be inserted in the second air inlet.

Preferably, the tube-shaped vaporizing core may comprise a tube-shaped porous element and a heating element, the tube-shaped porous element may serve to absorb, store, and transfer the vaporizing liquid, the tube-shaped porous element may be provided at a center with a tube hole, and the heating element may be provided on an inner wall of the tube hole, and the heating element may be connected at two ends with a positive lead and a negative lead.

Preferably, the tube-shaped porous element may be formed by a tube-shaped porous ceramic body.

Preferably, the heating element may be formed by an electric heating wire or an electric heating plate tightly abutting on or nested in an inner wall of the tube hole of the tube-shaped porous element.

Preferably, the hollow-out vaporizing cover part may consist of a connecting portion at an upper portion, a cover body at a middle portion, and a connecting base at a lower portion, wherein the connecting portion may be provided inside with a connecting hole, an upper portion of the connecting hole may be sleeved on a lower end of the mouthpiece tube, and a lower portion of the connecting hole may be sleeved on an upper end of the tube-shaped vaporizing core, a sealing sleeve may be further arranged between an inner wall of the connecting hole, the mouthpiece tube, and the tube-shaped vaporizing core, wherein the connecting base may comprise an outer ring and an inner ring, an outer wall of the outer ring may be fitted to an inner wall of the vaporizer housing, the cover body may be connected with the connecting portion and the inner ring of the connecting base, and the cover body may be hollowed out to define the opening.

Preferably, the mouthpiece end of the vaporizer housing may be provided with a groove extending downwards, the mouthpiece tube may be located at a center of the groove, and a vertical wall of the groove may be provided with several bulges protruding inwards.

Preferably, a mouthpiece sealing component may be further arranged between the mouthpiece end of the vaporizer housing and the mouthpiece cap, the mouthpiece sealing component may be provided with outlets at a corresponding position on two sides above the groove, and an upper end surface of the mouthpiece sealing component may be further provided with several protrusions for abutting against an inner wall of an upper end of the mouthpiece housing.

Preferably, positioning studs may be further provided on two sides of the mouthpiece end of the vaporizer housing, the mouthpiece sealing component may be provided with positioning holes at a position corresponding to the positioning studs, and the positioning holes may be fitted with the positioning studs.

Preferably, a mouthpiece groove may be defined by a hole wall which extends downwards to define the mouthpiece hole and an inner wall of an upper end of the mouthpiece cap, and a liquid absorbing component may be disposed in the mouthpiece groove.

### Advantages of the disclosure

### Advantages

As the vaporizer with the hollow-out vaporizing cover part is provided with the hollow-out vaporizing cover part having a cavity, and the middle wall portion is hollowed out to define several openings, the cavity is open to communicate with the liquid storage chamber through the openings, and the middle outer wall of the tube-shaped vaporizing core is entirely exposed to the cavity. In such a case, the vaporizing liquid inside the liquid storage chamber can freely flow into the cavity, and the middle outer wall of the tube-shaped vaporizing core can be fully immersed in the vaporizing liquid in the cavity without obstruction. Thus, during operation of the tube-shaped vaporizing core, quick feeding and absorbing of the vaporizing liquid can be achieved, thereby avoiding dry-heating caused by the lack of the liquid. Consequently, the amount of vapor that can enter the user's mouth can be increased, and user experience to vape can be improved.

### BRIEF DESCRIPTION OF THE DRAWINGS

### Description of the drawings

FIG.1 is an exploded perspective structural view of a vaporizer according to an embodiment of the disclosure;
FIG.2 is a front sectional view of a vaporizer according to an embodiment of the disclosure;
FIG.3 is a side sectional view of a vaporizer according to an embodiment of the disclosure;
FIG.4 is a perspective view of a mouthpiece cap of a vaporizer according to an embodiment of the disclosure;
FIG.5 is a sectional view of a vaporizer housing of a vaporizer according to an embodiment of the disclosure;
FIG.6 is a perspective view of a hollow-out vaporizing cover part according to an embodiment of the disclosure;
FIG.7 is a sectional view of a hollow-out vaporizing cover part according to an embodiment of the disclosure;
FIG.8 is a bottom view of a hollow-out vaporizing cover part according to an embodiment of the disclosure;
FIG.9 is a perspective view of a sealing sleeve according to an embodiment of the disclosure;
FIG.10 is a perspective view of a tube-shaped vaporizing core according to an embodiment of the disclosure;
FIG.11 is a sectional view of a tube-shaped vaporizing core according to an embodiment of the disclosure;
FIG.12 is a sectional view of a vaporizer housing according to an embodiment of the disclosure;
FIG.13 is a top view of a vaporizer housing according to an embodiment of the disclosure;
FIG.14 is a perspective view illustrating a mouthpiece sealing component according to an embodiment of the disclosure;
FIG.15 is a perspective view illustrating a mouthpiece sealing component according to an embodiment of the disclosure, being inverted;
FIG.16 is a sectional view of a mouthpiece sealing component according to an embodiment of the disclosure;
FIG.17 is a perspective view illustrating a liquid absorbing component according to an embodiment of the disclosure;
FIG.18 is a perspective view illustrating a seal seat according to an embodiment of the disclosure;
FIG.19 is a perspective view illustrating a seal seat according to an embodiment of the disclosure, being inverted;
FIG.20 is a perspective view illustrating a base according to an embodiment of the disclosure.

### DETAILED DESCRIPTION OF ILLUSTRATED EMBODIMENTS

### Preferred embodiments of the disclosure

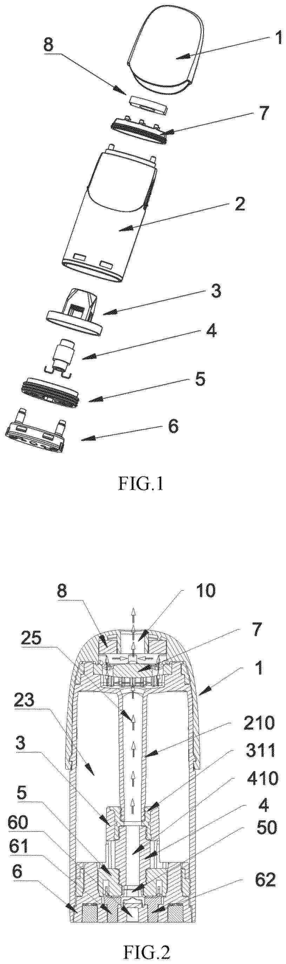

The vaporizer with the hollow-out vaporizing cover part of the disclosure can be connected with a battery part to constitute an electronic vaporizing device. For convenience of description, the mouthpiece cap 1 of the vaporizer with the hollow-out vaporizing cover part is vertically disposed to faces upwards, as shown in FIG.2. The terms, such as "upper", "lower", "upper portion", "lower portion", "upper end", "lower end", "upper surface", "lower surface", "upwards", "downwards" as used herein for illustrating the components, refer to position and orientation relationships when the vaporizer with the hollow-out vaporizing cover part is vertically disposed to face upwards.

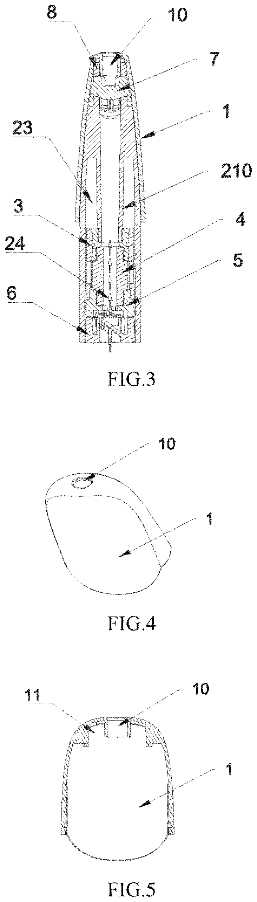

The vaporizer according to the embodiment has a working principle as follows:
Referring to FIGs.2 and 3, during operation of the vaporizer according to the embodiment, the vaporizing liquid stored in the liquid storage chamber 23 can freely flow to the cavity 30 through the opening 320 of the hollow-out vaporizing cover part 3. As the entire periphery of the outer wall of the tube-shaped vaporizing core is exposed to the cavity 30, the vaporizing liquid in the cavity 30 can be sufficiently fed to the tube-shaped vaporizing core 4. The heating element 42 heats and vaporizes the vaporizing liquid in the tube-shaped porous element 41, and the vapor can be produced in the tube hole 410 when the vaporizing liquid is vaporized. When a user vapes from the mouthpiece hole 10, a negative pressure is generated in 33 the tube hole 410 of the tube-shaped vaporizing core 4, which enables the air to enter through the first air inlet 60 of the liquid storage housing 2 and then enter the tube hole 410 of the tube-shaped vaporizing core through the second air inlet 50. Then, the vapor produced in the tube hole 410 can be driven by the incoming airflow and then flow upwards along with the airflow. They flow from the mouthpiece tube 210 to the groove 211 and then flow to the two sides. After that, they flow through the outlets 70 on two sides of the mouthpiece sealing component 7 and then flow to the center, i.e., the gap between the protrusions 71 of the mouthpiece sealing component, and finally flow out through the mouthpiece hole 10. The series of arrows as shown in FIGs.2 and 3 indicate the directions in which the air enters and the vapor is produced and flows out.

### Embodiments

### Embodiments of the disclosure

The disclosure will be further explained in detail with reference to the drawings.

### Embodiment 1

Referring to FIGs.1-8, the vaporizer with the hollow-out vaporizing cover part of the disclosure comprises a mouthpiece cap 1 and a vaporizer housing 2, which are connected with each other. Herein, the mouthpiece cap 1 is provided at its center with a mouthpiece hole 10, the vaporizer housing 2 has a mouthpiece end 21 and an opening end 22, the mouthpiece cap 1 is sleeved on the mouthpiece end 21 of the vaporizer housing, and the mouthpiece end 21 is closed and connected at its center with a mouthpiece tube 210 which extends downwards into the vaporizer housing. Inside the vaporizer housing 2, a hollow-out vaporizing cover part 3, a tube-shaped vaporizing core 4, a seal seat 5 and a base 6 are arranged in such order from top to bottom. The base 6 is sleeved on the opening end 22 of the vaporizer housing, and the base 6 is provided at its center with a first air inlet 60. The seal seat 5 is disposed between the hollow-out vaporizing cover part 3 and the base 6. The seal seat 5 is provided at its center with a second air inlet 50 which is in communication with the first air inlet 50, and a lower end of the tube-shaped vaporizing core 4 is inserted in the second air inlet 50.

The hollow-out vaporizing cover part 3 is hood shaped and has an upper end connected with a lower end of the mouthpiece tube 210. The lower end of the hollow-out vaporizing cover part 3 is open and is engaged with the seal seat 5 on the base. In such a case, a liquid storage chamber 23 for storing the vaporizing liquid is defined by the cavity between the inner wall of the vaporizer housing 2, the outer wall of the mouthpiece tube 210, and the upper surface of the hollow-out vaporizing cover part 3.

The hollow-out vaporizing cover part 3 is provided inside with a cavity 30. The middle wall portion of the hollow-out vaporizing cover part 3 is hollowed out to define several openings 320, and the cavity 30 is open to communicate with the liquid storage chamber 23 through the openings 320. The tube-shaped vaporizing core 4 is vertically arranged in the cavity 30 in such a manner that an upper end of the tube-shaped vaporizing core 4 is connected with the hollow-out vaporizing cover part 3 and in communication with the mouthpiece tube 210, a lower end of the tube-shaped vaporizing core 4 is in communication with the first air inlet 60, and the middle outer wall of the tube-shaped vaporizing core 4 is entirely exposed to the cavity 30. In such a case, the vaporizing liquid inside the liquid storage chamber 23 can freely flow into the cavity 30, and the middle outer wall of the tube-shaped vaporizing core 4 can be fully immersed in the vaporizing liquid in the cavity 30 without obstruction. Thus, during operation of the tube-shaped vaporizing core 4, quick feeding and absorbing of the vaporizing liquid can be achieved, thereby avoiding dry-heating caused by the lack of the liquid.

Referring to FIGs.2 and 3, the lower end of the tube-shaped vaporizing core 4 is sleeved in the second air inlet 50 which is provided at the center of the seal seat 5. The bottom surface of the seal seat 5 is sealingly connected with the upper end surface of the base 6, and the outer wall of the seal seat 5 is sealingly fitted to the inner wall of the vaporizer housing 2.

Referring to FIGs.2 and 3, the first air inlet 60, the second air inlet 50, the tube hole 410 of the tube-shaped vaporizing core, the mouthpiece tube 210, and the mouthpiece hole 10 are in communication in such order to define airflow paths 24, 25, and the airflow paths 24, 25 are sealed and separated from the liquid storage chamber 23 by several sealing components. Herein, the first air inlet 60, the second air inlet 50, the tube hole 410 of the tube-shaped porous element are in communication to define the airflow path 24. The tube hole 410, the mouthpiece tube 210, the mouthpiece hole 10 are in communication in such order to define the airflow path 25.

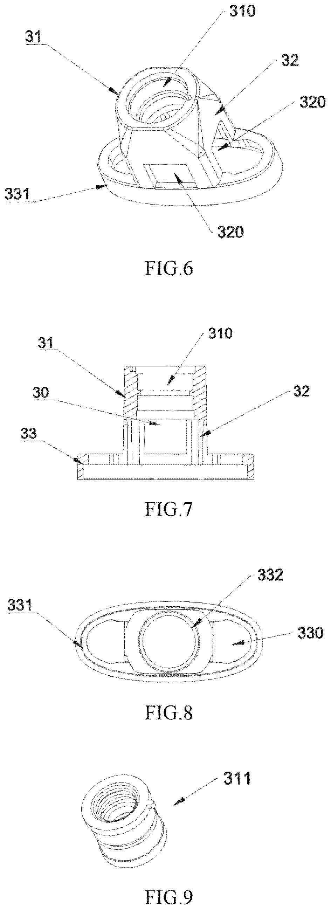

Referring to FIGs.6-9, the hollow-out vaporizing cover part 3 consists of a connecting portion 31 at the upper portion, a cover body 32 at the middle portion, and a connecting base 33 at the lower portion. The connecting portion 31 is provided inside with a connecting hole 310, wherein the upper portion of the connecting hole 310 is sleeved on the lower end of the mouthpiece tube 210, and the lower portion of the connecting hole 310 is sleeved on the upper end of the tube-shaped vaporizing core 4. Further, a sealing sleeve 311 is arranged between the inner wall of the connecting hole 310, the mouthpiece tube 210, and the tube-shaped vaporizing core 4. The connecting base 33 comprises an outer ring 331 and an inner ring 332. The outer wall of the outer ring 331 is fitted to the inner wall of the vaporizer housing 2, and the lower end surface of the connecting base 33 is connected with the upper end surface of the seal seat 5. The connecting portion 31 is connected and fixed with the inner ring 332 of the connecting base by the cover body 32. As the cover body 32 is hollowed out to define the openings 320, the middle outer wall of the tube-shaped vaporizing core 4 is entirely exposed to the cavity. Further, notches 330 are provided at two sides of the inner ring 332, and between the outer ring 331 and the inner ring 332, respectively. The notches 330 can facilitate the filling of the vaporizing liquid into the liquid storage chamber 23 during manufacturing.

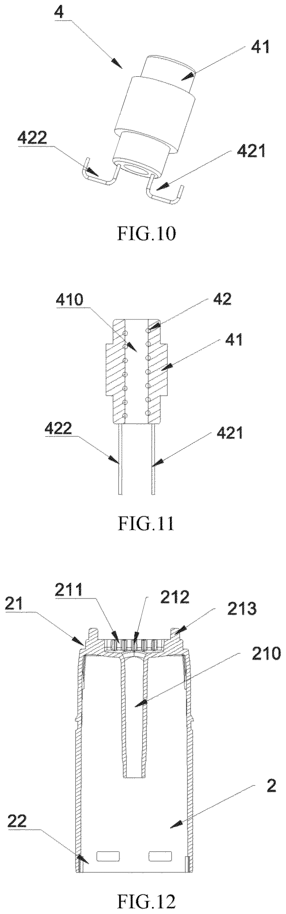

Referring to FIGs.10 and 11, the tube-shaped vaporizing core 4 comprises the tube-shaped porous element 41 and the heating element 42. The tube-shaped porous element 41 serves to absorb, store, and transfer the vaporizing liquid. The tube-shaped porous element 41 is provided with the tube hole 410, and the heating element 42 is provided on the inner wall of the tube hole 410. The heating element 42 is configured to generate heat when powered on, and the heating element 42 is connected at two ends with a positive lead 421 and a negative lead 422. In the embodiment, the tube-shaped porous element 41 is formed by tube-shaped porous ceramic body. The heating element 42 is formed by electric heating wires or electric heating plates tightly abutting on or nested in the inner wall of the tube hole 410 of the tube-shaped porous element.

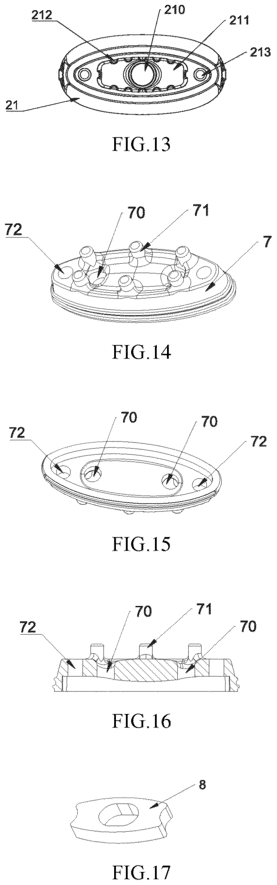

Referring to FIGs.12 and 13, the mouthpiece end 21 of the vaporizer housing is provided with a groove 211 extending downwards, and the mouthpiece tube 210 is located at the center of the groove 211. The vertical wall of the groove 211 is provided with several bulges 212 protruding inwards to define a curved path, which constitutes a portion of the vapor outlet path 25. When the vapor produced during vaping flows through the curved path from the mouthpiece tube 210 in middle to the two sides, the vapor can be impeded to reduce flow rate to a certain extent, such that the droplets contained in the vapor can be condensed between the bulges 212 and prevented from flowing out and leading to bad user experience.

Referring to FIGs.1-3 and 14-16, a mouthpiece sealing component 7 is further arranged between the mouthpiece end 21 of the vaporizer housing and the mouthpiece cap 1. The mouthpiece sealing component 7 is provided with outlets 70 at a corresponding position on two sides above the groove 211. The upper end surface of the mouthpiece sealing component 7 is further provided with several protrusions 71 for abutting against the inner wall of the upper end of the mouthpiece housing 2. The gap formed between the protrusions 71 constitutes a portion of the vapor outlet path 25.

Further, positioning studs 213 are provided on two sides of the mouthpiece end 21 of the vaporizer housing, the mouthpiece sealing component 7 is provided with positioning holes 72 at a position corresponding to the positioning studs 213, and the positioning holes 72 are fitted with the positioning studs 213. The alignment of the positioning holes 72 and the positioning studs 213 can ensure accurate mounting of the mouthpiece sealing component 7, and meanwhile avoid loosen or displacement of the mouthpiece sealing component 7 which may result in leakage and failure of vaping. The mouthpiece sealing component 7 may be made of soft silicone material.

Referring to FIGs.1-5 and 17, a mouthpiece groove 11 is defined by the hole wall which extends downwards to define the mouthpiece hole 10 and the inner wall of the upper end of the mouthpiece cap 1, and a liquid absorbing component 8 is disposed in the mouthpiece groove 11. The liquid absorbing component 8 serves to absorb the droplets which are not vaporized and condensed in the vapor, thereby preventing the droplets from entering the user's mouth to result in bad taste and poor user experience. The liquid absorbing component 8 is made of absorbent materials such as a sponge and organic cotton.

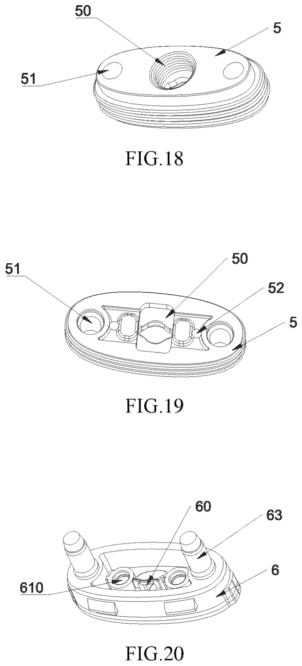

Referring to FIGs.18 and 19, the seal seat 5 is made of soft silicone material and has high temperature resistance. The seal seat 5 is provided with the second air inlet 50 at its center, liquid filling through-holes 51 on the two sides, and blind holes 52 for accommodating the positive lead 421 and the negative lead 422 at the bottom. The positive lead 421 and the negative lead 422 respectively pass through the seal seat 5 and then are bent, with their tips being accommodated in the blind holes 52.

Referring to FIG.20, the base 6 is provided with two electrode through-holes 610, and a positive electrode 61 and a negative electrode 62 are respectively placed in the electrode through-holes 610. The positive electrode 61 and the negative electrode 62 are respectively connected with and abutting against the bent portions of the positive leads 421 and the negative leads 422 which extend out from the seal seat 5. The base 6 is further provided with a filling plunger 63. Prior to the mounting of the base, the vaporizing liquid may be filled into the liquid storage chamber 23 through the liquid filling through-holes 51. Then, the base 6 may be mounted, such that the filling plunger 63 closes the liquid filling through-hole 51 to avoid leakage.

### Industrial applicability

All the above are merely preferred embodiments of the disclosure. The present invention is intended to cover all equivalent arrangements and modifications derived from the claims of the present invention.

## Claims

1. A vaporizer with a hollow-out vaporizing cover part, **characterized by** comprising a mouthpiece cap (1) and a vaporizer housing (2), wherein the mouthpiece cap (1) is provided at an upper end with a mouthpiece hole (10), the vaporizer housing (2) has a mouthpiece end (21) and an opening end (22), the mouthpiece cap (1) is sleeved on the mouthpiece end (21) of the vaporizer housing (2), and the mouthpiece end (21) of the vaporizer housing is closed and is arranged at a center with a mouthpiece tube (210) extending downwards into the vaporizer housing (2), and a hollow-out vaporizing cover part (3), a tube-shaped vaporizing core (4), and a base (6) are arranged inside the vaporizer housing (2);
the base (6) is sleeved on the opening end (22) of the vaporizer housing (2), and the base (6) is provided at a center with a first air inlet (60);
the hollow-out vaporizing cover part (3) has an upper end connected with a lower end of the mouthpiece tube (210), a lower end of the hollow-out vaporizing cover part (3) is open and is engaged on the base (6), and a liquid storage chamber (23) for storing the vaporizing liquid is defined by a cavity formed between an inner wall of the vaporizer housing (2), an outer wall of the mouthpiece tube (210), and an upper surface of the hollow-out vaporizing cover part (3);
the hollow-out vaporizing cover part (3) is provided inside with a cavity (30), a middle wall portion of the hollow-out vaporizing cover part (3) is hollowed out to define an opening (320), and the cavity (30) is open to communicate with the liquid storage chamber (23) through the opening (320), the tube-shaped vaporizing core (4) is vertically arranged in the cavity (30) in such a manner that an upper end of the tube-shaped vaporizing core (4) is connected with the hollow-out vaporizing cover part (3) and in communication with the mouthpiece tube (210), a lower end thereof is in communication with the first air inlet (60), and the middle outer wall of the tube-shaped vaporizing core (4) is entirely exposed to the cavity (30); and
the first air inlet (60), a tube hole (410) of the tube-shaped vaporizing core (4), the mouthpiece tube (210), and the mouthpiece hole (10) are in communication in such order to define airflow paths (24, 25) which are sealed and separated from the liquid storage chamber (23).

2. The vaporizer with a hollow-out vaporizing cover part according to claim 1, wherein a seal seat (5) is further disposed between the tube-shaped vaporizing core (4) and the base (6), the seal seat (5) seals the liquid storage chamber (23), the seal seat (5) is provided at a center with a second air inlet (50) in communication with the first air inlet (60), and a lower end of the tube-shaped vaporizing core (4) is inserted in the second air inlet (50).

3. The vaporizer with a hollow-out vaporizing cover part according to claim 1, wherein the tube-shaped vaporizing core (4) comprises a tube-shaped porous element (41) and a heating element (42), the tube-shaped porous element (41) serves to absorb, store, and transfer the vaporizing liquid, the tube-shaped porous element (4) is provided at a center with a tube hole (410), and the heating element (42) is provided on an inner wall of the tube hole (410), and the heating element (42) is connected at two ends with a positive lead (421) and a negative lead (422).

4. The vaporizer with a hollow-out vaporizing cover part according to claim 3, wherein the tube-shaped porous element (41) is formed by a tube-shaped porous ceramic body.

5. The vaporizer with a hollow-out vaporizing cover part according to claim 3, wherein the heating element (42) is formed by an electric heating wire or an electric heating plate tightly abutting on or nested in an inner wall of the tube hole (410) of the tube-shaped porous element.

6. The vaporizer with a hollow-out vaporizing cover part according to claim 1, wherein the hollow-out vaporizing cover part (3) consists of a connecting portion (31) at an upper portion, a cover body (32) at a middle portion, and a connecting base (33) at a lower portion, wherein the connecting portion (31) is provided inside with a connecting hole (310), an upper portion of the connecting hole (310) is sleeved on a lower end of the mouthpiece tube (210), and a lower portion of the connecting hole (310) is sleeved on an upper end of the tube-shaped vaporizing core (4), wherein a sealing sleeve (311) is further arranged between an inner wall of the connecting hole (310), the mouthpiece tube (210), and the tube-shaped vaporizing core (4), wherein the connecting base (33) comprises an outer ring (331) and an inner ring (332), an outer wall of the outer ring (331) is fitted to an inner wall of the vaporizer housing (2), the cover body (32) is connected with the connecting portion (31) and the inner ring (332) of the connecting base, and the cover body (32) is hollowed out to define the opening (320).

7. The vaporizer with a hollow-out vaporizing cover part according to claim 1, wherein the mouthpiece end (21) of the vaporizer housing is provided with a groove (211) extending downwards, the mouthpiece tube (210) is located at a center of the groove (211), and a vertical wall of the groove (211) is provided with several bulges (212) protruding inwards.

8. The vaporizer with a hollow-out vaporizing cover part according to claim 7, wherein a mouthpiece sealing component (7) is further arranged between the mouthpiece end (21) of the vaporizer housing and the mouthpiece cap (1), the mouthpiece sealing component (7) is provided with outlets (70) at a corresponding position on two sides above the groove (211), and an upper end surface of the mouthpiece sealing component (7) is further provided with several protrusions (71) for abutting against an inner wall of an upper end of the mouthpiece housing (2).

9. The vaporizer with a hollow-out vaporizing cover part according to claim 8, wherein positioning studs (213) are further provided on two sides of the mouthpiece end (21) of the vaporizer housing, the mouthpiece sealing component (7) is provided with positioning holes (72) at a position corresponding to the positioning studs (213), and the positioning holes (72) are fitted with the positioning studs (213).

10. The vaporizer with a hollow-out vaporizing cover part according to claim 1, wherein a mouthpiece groove (11) is defined by a hole wall which extends downwards to define the mouthpiece hole (10) and an inner wall of an upper end of the mouthpiece cap (1), and a liquid absorbing component (8) is disposed in the mouthpiece groove (11).
